# EUROPEAN PATENT APPLICATION

(11) **EP 4 738 050 A1**
(43) Date of publication of application: **06.05.2026**
(21) Application number: 24849346.2
(22) Date of filing: 05.06.2024
(51) Int. Cl.: G06F 1/16, H05K 5/02, G06F 3/01, H05K 1/14

(54) **WEARABLE DEVICE COMPRISING INSULATION STRUCTURE**

(30) Priority: 02.08.2023 KR 20230101320; 24.08.2023 KR 20230111587
(71) Applicant: Samsung Electronics Co., Ltd., Suwon-si, Gyeonggi-do 16677 (KR)
(72) Inventor: LEE, Hosoon, Suwon-si, Gyeonggi-do 16677 (KR); BAE, Yosep, Suwon-si, Gyeonggi-do 16677 (KR); SONG, Kyunghwan, Suwon-si, Gyeonggi-do 16677 (KR); CHOI, Hyunsuk, Suwon-si, Gyeonggi-do 16677 (KR)
(74) Representative: HGF
(86) International application number: PCT/KR2024/007725
(87) International publication number: WO 2025/028775

(57) **Abstract**

A wearable device according to one embodiment may comprise a housing comprising: a first surface facing a part of the body of a user when the wearable device is worn on the user; and a second surface opposite to the first surface. The wearable device may comprise an electronic component between the first surface and the second surface. The wearable device may comprise a heat insulation structure inside the housing, that is at least partially disposed between the electronic component and the first surface and comprising at least one air gap.

## Description

### [Technical Field]

Various embodiments to be described later relate to a wearable device including a heat insulation structure.

### [Background Art]

A wearable device may be used while being worn on a part of a body of a user. The wearable device may be provided as various types of products. For example, the wearable device may include a ring-shaped device for the user to be worn on the part of the body of the user. The wearable device may include various electronic components to meet a demand of the user. As electronic components disposed in the wearable device perform an operation for responding to a request of the user, heat may be generated in the miniaturized wearable device.

The above-described information may be provided as a related art for the purpose of helping understanding of the present disclosure. No argument or decision is made as to whether any of the above description may be applied as a prior art related to the present disclosure.

### [Disclosure]

### [Technical Solution]

According to an embodiment, a wearable device may comprise a housing including a first surface facing a part of a body of a user while the wearable device is worn by the user, and a second surface opposite to the first surface. The wearable device may comprise an electronic component disposed between the first surface and the second surface. The wearable device may comprise a heat insulation structure in the housing, disposed at least partially between the electronic component and the first surface, and including at least one air gap for reducing heat transfer from the electronic component to the first surface.

According to an embodiment, a wearable device may comprise a housing including a first frame including a first surface facing a part of a body of a user while the wearable device is worn by the user, a second frame including a second surface opposite to the first surface , and a third frame disposed between the first frame and the second frame. The wearable device may comprise an electronic component disposed in the third frame. The third frame may include a third surface facing toward the first frame. The third frame may include a heat insulation structure, disposed at least partially between the electronic component and the first frame, and including at least one groove recessed from the third surface toward the electronic component, and at least one air gap formed by the at least one groove for reducing heat transfer from the electronic component to the first surface.

### [Description of the Drawings]

FIG. 1 is a block diagram of an electronic device in a network environment according to an embodiment.
FIG. 2 illustrates an exemplary electronic device.
FIG. 3A is a cross-sectional view of an exemplary electronic device.
FIG. 3B illustrates a part of an exemplary electronic device.
FIGS. 4A, 4B, and 4C are partial cross-sectional views of an exemplary electronic device.
FIGS. 5A, 5B, 5C, and 5D are partial cross-sectional views of an exemplary electronic device.
FIGS. 6A and 6B are partial cross-sectional views of an exemplary electronic device.
FIG. 7 illustrates a portion of a manufacturing process of an exemplary electronic device.
FIG. 8 illustrates a portion of a manufacturing process of an exemplary electronic device.

### [Mode for Invention]

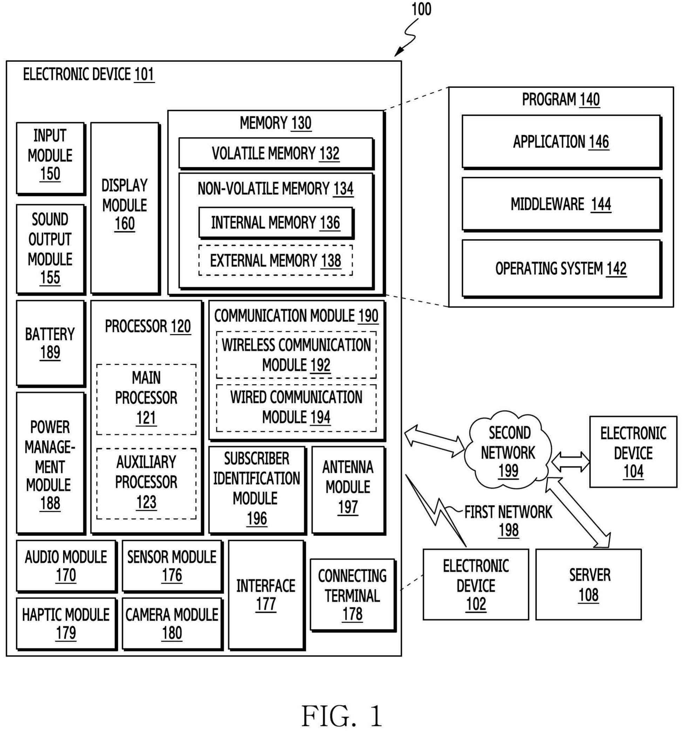

FIG. 1 is a block diagram illustrating an electronic device 101 in a network environment 100 according to various embodiments.

Referring to FIG. 1, the electronic device 101 in the network environment 100 may communicate with an electronic device 102 via a first network 198 (e.g., a short-range wireless communication network), or at least one of an electronic device 104 or a server 108 via a second network 199 (e.g., a long-range wireless communication network). According to an embodiment, the electronic device 101 may communicate with the electronic device 104 via the server 108. According to an embodiment, the electronic device 101 may include a processor 120, memory 130, an input module 150, a sound output module 155, a display module 160, an audio module 170, a sensor module 176, an interface 177, a connecting terminal 178, a haptic module 179, a camera module 180, a power management module 188, a battery 189, a communication module 190, a subscriber identification module(SIM) 196, or an antenna module 197. In some embodiments, at least one of the components (e.g., the connecting terminal 178) may be omitted from the electronic device 101, or one or more other components may be added in the electronic device 101. In some embodiments, some of the components (e.g., the sensor module 176, the camera module 180, or the antenna module 197) may be implemented as a single component (e.g., the display module 160).

The processor 120 may execute, for example, software (e.g., a program 140) to control at least one other component (e.g., a hardware or software component) of the electronic device 101 coupled with the processor 120, and may perform various data processing or computation. According to an embodiment, as at least part of the data processing or computation, the processor 120 may store a command or data received from another component (e.g., the sensor module 176 or the communication module 190) in volatile memory 132, process the command or the data stored in the volatile memory 132, and store resulting data in non-volatile memory 134. According to an embodiment, the processor 120 may include a main processor 121 (e.g., a central processing unit (CPU) or an application processor (AP)), or an auxiliary processor 123 (e.g., a graphics processing unit (GPU), a neural processing unit (NPU), an image signal processor (ISP), a sensor hub processor, or a communication processor (CP)) that is operable independently from, or in conjunction with, the main processor 121. For example, when the electronic device 101 includes the main processor 121 and the auxiliary processor 123, the auxiliary processor 123 may be adapted to consume less power than the main processor 121, or to be specific to a specified function. The auxiliary processor 123 may be implemented as separate from, or as part of the main processor 121.

The auxiliary processor 123 may control at least some of functions or states related to at least one component (e.g., the display module 160, the sensor module 176, or the communication module 190) among the components of the electronic device 101, instead of the main processor 121 while the main processor 121 is in an inactive (e.g., sleep) state, or together with the main processor 121 while the main processor 121 is in an active state (e.g., executing an application). According to an embodiment, the auxiliary processor 123 (e.g., an image signal processor or a communication processor) may be implemented as part of another component (e.g., the camera module 180 or the communication module 190) functionally related to the auxiliary processor 123. According to an embodiment, the auxiliary processor 123 (e.g., the neural processing unit) may include a hardware structure specified for artificial intelligence model processing. An artificial intelligence model may be generated by machine learning. Such learning may be performed, e.g., by the electronic device 101 where the artificial intelligence is performed or via a separate server (e.g., the server 108). Learning algorithms may include, but are not limited to, e.g., supervised learning, unsupervised learning, semi-supervised learning, or reinforcement learning. The artificial intelligence model may include a plurality of artificial neural network layers. The artificial neural network may be a deep neural network (DNN), a convolutional neural network (CNN), a recurrent neural network (RNN), a restricted Boltzmann machine (RBM), a deep belief network (DBN), a bidirectional recurrent deep neural network (BRDNN), deep Q-network or a combination of two or more thereof but is not limited thereto. The artificial intelligence model may, additionally or alternatively, include a software structure other than the hardware structure.

The memory 130 may store various data used by at least one component (e.g., the processor 120 or the sensor module 176) of the electronic device 101. The various data may include, for example, software (e.g., the program 140) and input data or output data for a command related thereto. The memory 130 may include the volatile memory 132 or the non-volatile memory 134.

The program 140 may be stored in the memory 130 as software, and may include, for example, an operating system (OS) 142, middleware 144, or an application 146.

The input module 150 may receive a command or data to be used by another component (e.g., the processor 120) of the electronic device 101, from the outside (e.g., a user) of the electronic device 101. The input module 150 may include, for example, a microphone, a mouse, a keyboard, a key (e.g., a button), or a digital pen (e.g., a stylus pen).

The sound output module 155 may output sound signals to the outside of the electronic device 101. The sound output module 155 may include, for example, a speaker or a receiver. The speaker may be used for general purposes, such as playing multimedia or playing record. The receiver may be used for receiving incoming calls. According to an embodiment, the receiver may be implemented as separate from, or as part of the speaker.

The display module 160 may visually provide information to the outside (e.g., a user) of the electronic device 101. The display module 160 may include, for example, a display, a hologram device, or a projector and control circuitry to control a corresponding one of the display, hologram device, and projector. According to an embodiment, the display module 160 may include a touch sensor adapted to detect a touch, or a pressure sensor adapted to measure the intensity of force incurred by the touch.

The audio module 170 may convert a sound into an electrical signal and vice versa. According to an embodiment, the audio module 170 may obtain the sound via the input module 150, or output the sound via the sound output module 155 or a headphone of an external electronic device (e.g., an electronic device 102) directly (e.g., wiredly) or wirelessly coupled with the electronic device 101.

The sensor module 176 may detect an operational state (e.g., power or temperature) of the electronic device 101 or an environmental state (e.g., a state of a user) external to the electronic device 101, and then generate an electrical signal or data value corresponding to the detected state. According to an embodiment, the sensor module 176 may include, for example, a gesture sensor, a gyro sensor, an atmospheric pressure sensor, a magnetic sensor, an acceleration sensor, a grip sensor, a proximity sensor, a color sensor, an infrared (IR) sensor, a biometric sensor, a temperature sensor, a humidity sensor, or an illuminance sensor.

The interface 177 may support one or more specified protocols to be used for the electronic device 101 to be coupled with the external electronic device (e.g., the electronic device 102) directly (e.g., wiredly) or wirelessly. According to an embodiment, the interface 177 may include, for example, a high definition multimedia interface (HDMI), a universal serial bus (USB) interface, a secure digital (SD) card interface, or an audio interface.

A connecting terminal 178 may include a connector via which the electronic device 101 may be physically connected with the external electronic device (e.g., the electronic device 102). According to an embodiment, the connecting terminal 178 may include, for example, an HDMI connector, a USB connector, an SD card connector, or an audio connector (e.g., a headphone connector).

The haptic module 179 may convert an electrical signal into a mechanical stimulus (e.g., a vibration or a movement) or electrical stimulus which may be recognized by a user via his tactile sensation or kinesthetic sensation. According to an embodiment, the haptic module 179 may include, for example, a motor, a piezoelectric element, or an electric stimulator.

The camera module 180 may capture a still image or moving images. According to an embodiment, the camera module 180 may include one or more lenses, image sensors, image signal processors, or flashes.

The power management module 188 may manage power supplied to the electronic device 101. According to an embodiment, the power management module 188 may be implemented as at least part of, for example, a power management integrated circuit (PMIC).

The battery 189 may supply power to at least one component of the electronic device 101. According to an embodiment, the battery 189 may include, for example, a primary cell which is not rechargeable, a secondary cell which is rechargeable, or a fuel cell.

The communication module 190 may support establishing a direct (e.g., wired) communication channel or a wireless communication channel between the electronic device 101 and the external electronic device (e.g., the electronic device 102, the electronic device 104, or the server 108) and performing communication via the established communication channel. The communication module 190 may include one or more communication processors that are operable independently from the processor 120 (e.g., the application processor (AP)) and supports a direct (e.g., wired) communication or a wireless communication. According to an embodiment, the communication module 190 may include a wireless communication module 192 (e.g., a cellular communication module, a short-range wireless communication module, or a global navigation satellite system (GNSS) communication module) or a wired communication module 194 (e.g., a local area network (LAN) communication module or a power line communication (PLC) module). A corresponding one of these communication modules may communicate with the external electronic device via the first network 198 (e.g., a short-range communication network, such as Bluetooth^{™}, wireless-fidelity (Wi-Fi) direct, or infrared data association (IrDA)) or the second network 199 (e.g., a long-range communication network, such as a legacy cellular network, a 5G network, a next-generation communication network, the Internet, or a computer network (e.g., LAN or wide area network (WAN)). These various types of communication modules may be implemented as a single component (e.g., a single chip), or may be implemented as multi components (e.g., multi chips) separate from each other. The wireless communication module 192 may identify and authenticate the electronic device 101 in a communication network, such as the first network 198 or the second network 199, using subscriber information (e.g., international mobile subscriber identity (IMSI)) stored in the subscriber identification module 196.

The wireless communication module 192 may support a 5G network, after a 4G network, and next-generation communication technology, e.g., new radio (NR) access technology. The NR access technology may support enhanced mobile broadband (eMBB), massive machine type communications (mMTC), or ultra-reliable and low-latency communications (URLLC). The wireless communication module 192 may support a high-frequency band (e.g., the mmWave band) to achieve, e.g., a high data transmission rate. The wireless communication module 192 may support various technologies for securing performance on a high-frequency band, such as, e.g., beamforming, massive multiple-input and multiple-output (massive MIMO), full dimensional MIMO (FD-MIMO), array antenna, analog beam-forming, or large scale antenna. The wireless communication module 192 may support various requirements specified in the electronic device 101, an external electronic device (e.g., the electronic device 104), or a network system (e.g., the second network 199). According to an embodiment, the wireless communication module 192 may support a peak data rate (e.g., 20Gbps or more) for implementing eMBB, loss coverage (e.g., 164dB or less) for implementing mMTC, or U-plane latency (e.g., 0.5ms or less for each of downlink (DL) and uplink (UL), or a round trip of 1ms or less) for implementing URLLC.

The antenna module 197 may transmit or receive a signal or power to or from the outside (e.g., the external electronic device) of the electronic device 101. According to an embodiment, the antenna module 197 may include an antenna including a radiating element composed of a conductive material or a conductive pattern formed in or on a substrate (e.g., a printed circuit board (PCB)). According to an embodiment, the antenna module 197 may include a plurality of antennas (e.g., array antennas). In such a case, at least one antenna appropriate for a communication scheme used in the communication network, such as the first network 198 or the second network 199, may be selected, for example, by the communication module 190 (e.g., the wireless communication module 192) from the plurality of antennas. The signal or the power may then be transmitted or received between the communication module 190 and the external electronic device via the selected at least one antenna. According to an embodiment, another component (e.g., a radio frequency integrated circuit (RFIC)) other than the radiating element may be additionally formed as part of the antenna module 197.

According to various embodiments, the antenna module 197 may form a mmWave antenna module. According to an embodiment, the mmWave antenna module may include a printed circuit board, an RFIC disposed on a first surface (e.g., the bottom surface) of the printed circuit board, or adjacent to the first surface and capable of supporting a designated high-frequency band (e.g., the mmWave band), and a plurality of antennas (e.g., array antennas) disposed on a second surface (e.g., the top or a side surface) of the printed circuit board, or adjacent to the second surface and capable of transmitting or receiving signals of the designated high-frequency band.

At least some of the above-described components may be coupled mutually and communicate signals (e.g., commands or data) therebetween via an inter-peripheral communication scheme (e.g., a bus, general purpose input and output (GPIO), serial peripheral interface (SPI), or mobile industry processor interface (MIPI)).

According to an embodiment, commands or data may be transmitted or received between the electronic device 101 and the external electronic device 104 via the server 108 coupled with the second network 199. Each of the electronic devices 102 or 104 may be a device of a same type as, or a different type, from the electronic device 101. According to an embodiment, all or some of operations to be executed at the electronic device 101 may be executed at one or more of the external electronic devices 102, 104, or 108. For example, if the electronic device 101 should perform a function or a service automatically, or in response to a request from a user or another device, the electronic device 101, instead of, or in addition to, executing the function or the service, may request the one or more external electronic devices to perform at least part of the function or the service. The one or more external electronic devices receiving the request may perform the at least part of the function or the service requested, or an additional function or an additional service related to the request, and transfer an outcome of the performing to the electronic device 101. The electronic device 101 may provide the outcome, with or without further processing of the outcome, as at least part of a reply to the request. To that end, a cloud computing, distributed computing, mobile edge computing (MEC), or client-server computing technology may be used, for example. The electronic device 101 may provide ultra low-latency services using, e.g., distributed computing or mobile edge computing. In another embodiment, the external electronic device 104 may include an internet-of-things (IoT) device. The server 108 may be an intelligent server using machine learning and/or a neural network. According to an embodiment, the external electronic device 104 or the server 108 may be included in the second network 199. The electronic device 101 may be applied to intelligent services (e.g., smart home, smart city, smart car, or healthcare) based on 5G communication technology or IoT-related technology.

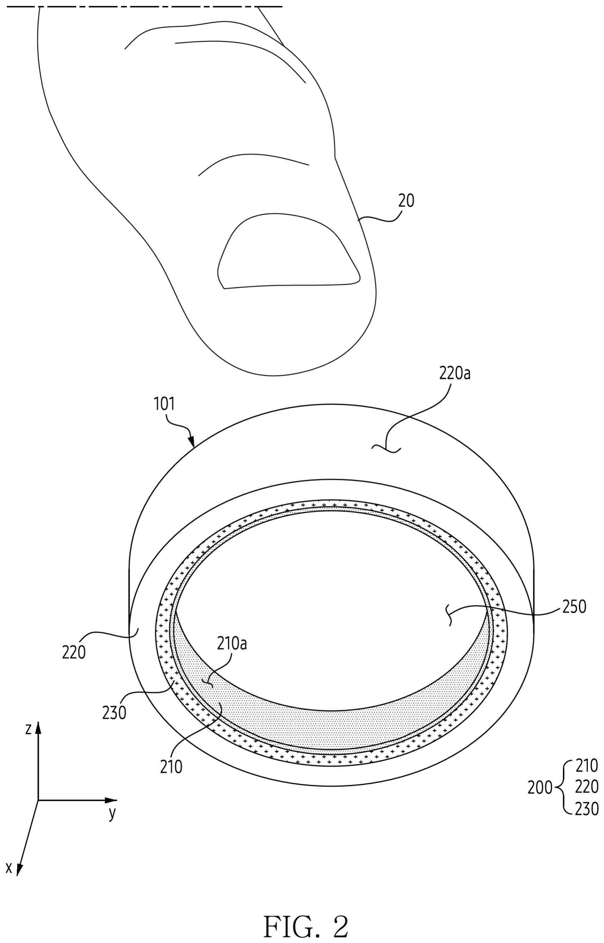

FIG. 2 illustrates an exemplary electronic device.

Referring to FIG. 2, an electronic device 101 may include a housing 200.

According to an embodiment, the electronic device 101 may be referred to as a wearable device that may be worn by a user. The user may mean a person who wears the electronic device 101. The electronic device 101 may be worn on a part 20 of a body of the user. For example, the electronic device 101 may be worn on the part 20 of the body of the user. For example, the electronic device 101 may be fastened to the part 20 of the body of the user. For example, the electronic device 101 may be detachable with respect to the part 20 of the body of the user.

For example, the electronic device 101 may be in contact with the part 20 of the body of the user by being worn by the user. For example, the electronic device 101 may be configured to obtain information related to the user through the part 20 of the body of the user by being worn by the user. For example, the electronic device 101 may provide the user with information indicating a state of the user, based on obtaining the information related to the user. For example, the electronic device 101 may be configured to display the information indicating the state of the user through a display module (e.g., the display module 160 of FIG. 1) of the electronic device 101 and/or an external electronic device (e.g., the electronic device 102 or the electronic device 104 of FIG. 1) connected to the electronic device 101, to provide the user with the information indicating the state of the user. However, it is not limited thereto.

For example, the part 20 of the body of the user on which the electronic device 101 is worn may be a finger of the user. For example, the housing 200 of the electronic device 101 may have a ring shape so that the electronic device 101 is worn on the finger of the user. However, it is not limited thereto. The electronic device 101 which may be referred to as a wearable device may have a shape corresponding to the part 20 of the body in order to be worn on the part 20 of the body of the user.

According to an embodiment, the housing 200 may include a first surface 210a facing the part 20 of the body of the user when the electronic device 101 is worn on the user, and a second surface 220a opposite to the first surface 210a.

For example, when the electronic device 101 is worn on the user, the first surface 210a may be at least partially in contact with the part 20 of the body of the user. For example, the first surface 210a may surround the part 20 of the body of the user on the electronic device 101 is worn. For example, the first surface 210a may cover the part 20 of the body of the user on which the electronic device 101 is worn. For example, the first surface 210a may be configured such that the electronic device 101 is fastened to the part 20 of the body by pressurizing the part 20 of the body of the user when the electronic device 101 is worn on the user. For example, the first surface 210a may be deformable by the part 20 of the body of the user. For example, the electronic device 101 may provide haptic technology to transmit the information related to the state of the user through the first surface 210a when the electronic device 101 is worn on the user.

For example, the second surface 220a may form an exterior of the electronic device 101 together with the first surface 210a. For example, the second surface 220a may form the ring-shaped housing 200 together with the first surface 210a. For example, the second surface 220a may be a surface spaced apart from the part 20 of the body of the user when the electronic device 101 is worn by the user. For example, when the electronic device 101 is worn on the user, the first surface 210a may be a surface closest to the part 20 of the body of the user. The second surface 220a opposite to the first surface 210a may be a surface farthest from the part 20 of the body of the user. For example, the first surface 210a may be referred to as an inner circumference surface of the housing 200. The second surface 220a opposite to the first surface 210a may be referred to as an outer circumference surface of the housing 200.

According to an embodiment, the housing 200 may further include a first frame 210, a second frame 220, and a third frame 230. The first frame 210 may define the first surface 210a. The second frame 220 may define the second surface 220a. The third frame 230 may be interposed between the first frame 210 and the second frame 220.

For example, the first frame 210 may be a part of the housing 200 including the first surface 210a. For example, the first frame 210 may be in contact with the part 20 of the body of the user while the electronic device 101 is worn on the user. For example, the first frame 210 may be pressurized by the part 20 of the body of the user.

For example, the second frame 220 may be a part of the housing 200 including the second surface 220a opposite to the first surface 210a. For example, it may surround the first frame 210. For example, the second frame 220 may be coupled with the first frame 210. For example, the second frame 220 may be attached to the first frame 210 through the third frame 230. For example, the second frame 220 may be spaced apart from the first frame 210 by the third frame 230.

For example, the third frame 230 may be disposed between the first frame 210 and the second frame 220. For example, the third frame 230 may fill a space between the first frame 210 and the second frame 220. For example, the third frame 230 may separate the first frame 210 and the second frame 220 at a designated interval by being interposed between the first frame 210 and the second frame 220. For example, the third frame 230 may be a part of the housing 200 including electronic components of the electronic device 101.

According to an embodiment, the second frame 220 may include at least one of metal and titanium. The third frame 230 may include at least one of silicon, epoxy, and acrylic. However, it is not limited thereto. By including the frames 210, 220, and 230 each including different materials, the housing 200 may provide a space for electronic components in the electronic device 101 and increase wearability of the user while the user wears the electronic device 101.

According to an embodiment, the electronic device 101 may further include a hole 250 formed by the first surface 210a through which the part 20 of the body of the user passes when the electronic device 101 is worn on the user. For example, the first surface 210a may define the hole 250. For example, when the electronic device 101 is worn on the user, the hole 250 may be penetrated by the part 20 of the body of the user. The electronic device 101 may be configured to be fastened to the part 20 of the body of the user when the user wears the electronic device 101.

According to the above-described embodiment, the electronic device 101 may provide the space for the electronic components in the electronic device 101 by including the housing 200 including the first frames 210, 220, and 230, and may increase the wearability of the user while the user wears the electronic device 101.

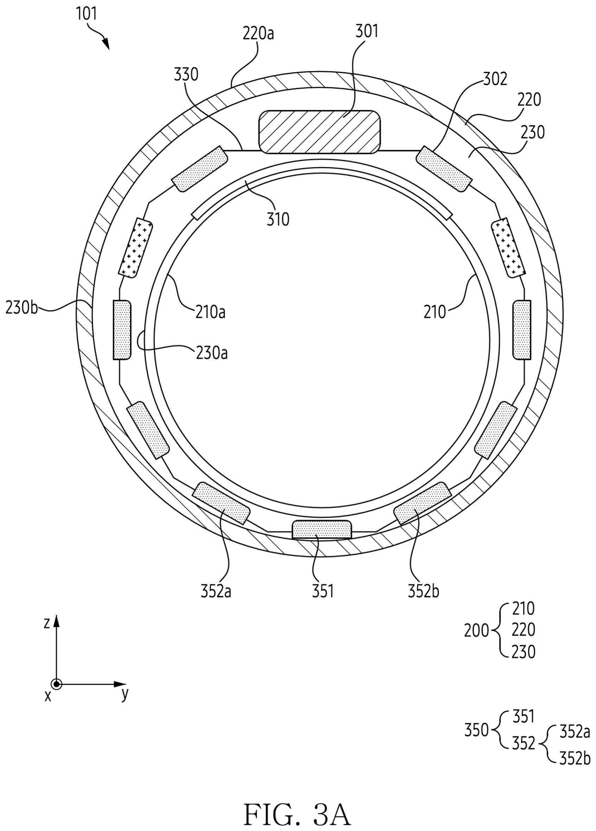

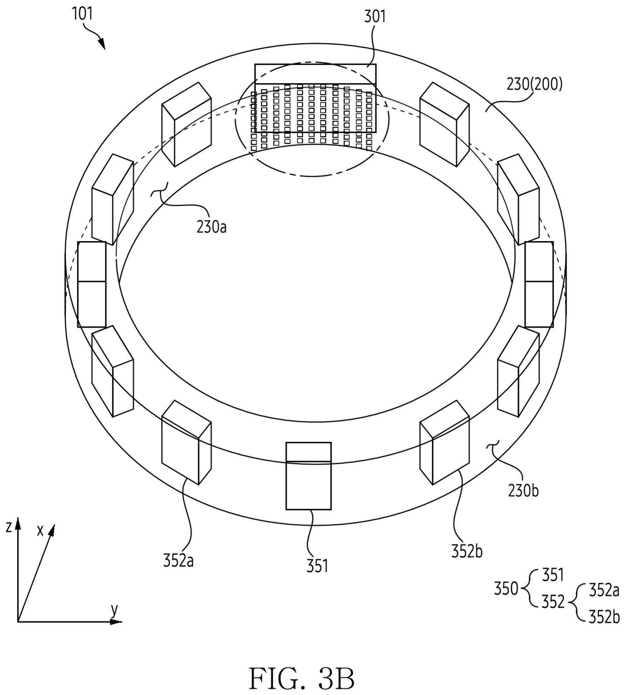

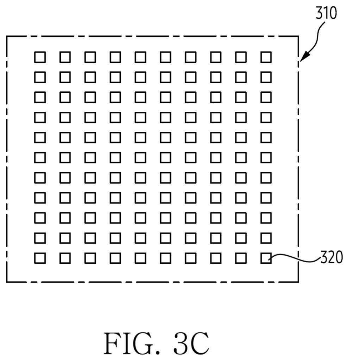

FIG. 3A is a cross-sectional view of an exemplary electronic device. FIG. 3B illustrates a part of an exemplary electronic device.

Referring to FIGS. 3A and 3B, an electronic device 101 may include a housing 200 including a first surface 210a and a second surface 220a which is opposite the first surface 210a, an electronic component 301, and a heat insulation structure 310. According to an embodiment, the housing 200 may further include a first frame 210, a second frame 220, and a third frame 230 interposed between the first frame 210 and the second frame 220.

Hereinafter, an overlapping description of a configuration having the same reference numerals as the above-described configuration will be omitted.

According to an embodiment, the electronic component 301 may be disposed between the first surface 210a and the second surface 220a of the housing 200. For example, the electronic component 301 may be disposed in the housing 200. For example, the electronic component 301 may emit heat as the electronic device 101 operates. At least a part of the heat may be transferred to a part of a body (e.g., the part 20 of the body of FIG. 2) of a user wearing the electronic device 101 through the first surface 210a of the housing 200. The electronic device 101 may require a structure for reducing heat transfer from the electronic component 301 to the first surface 210a configured to face the part 20 of the body of the user.

According to an embodiment, the electronic component 301 may be disposed in the third frame 230 between the first frame 210 and the second frame 220. For example, the third frame 230 may be a part including a plurality of electronic components of the electronic device 101 including the electronic component 301. For example, the third frame 230 may form a space for the electronic component 301 of the electronic device 101. For example, the heat emitted from the electronic component 301 may be transferred to the first frame 210 and/or the second frame 220 which are coupled with the third frame 230 through the third frame 230.

According to an embodiment, the third frame 230 may include a third surface 230a facing toward the first frame 210, and a fourth surface 230b opposite the third surface 230a and facing toward the second frame 220. For example, the electronic component 301 in the electronic device 101 may be disposed between the third surface 230a and the fourth surface 230b opposite the third surface 230a of the third frame 230. For example, the third surface 230a may be attached to the first frame 210. The fourth surface 230b opposite to the third surface 230a may be attached to the second frame 220.

According to an embodiment, the heat insulation structure 310 may be at least partially disposed between the electronic component 301 and the first surface 210a of the housing 200. The heat insulation structure 310 may include at least one air gap 320 for reducing heat transfer from the electronic component 301 to the first surface 210a.

For example, the heat insulation structure 310 may be interposed between the electronic component 301 and the first surface 210a. For example, the heat insulation structure 310may be positioned in a direction in which the electronic component 301 faces toward the first surface 210a with respect to the electronic component 301. For example, the heat insulation structure 310 may be disposed above the first surface 210a. For example, the heat insulation structure 310 may be disposed along the first surface 210a. For example, at least a part of the heat insulation structure 310 may face the electronic component 301 and be spaced apart from the electronic component 301. For example, the heat insulation structure 310 may face at least a part of the first surface 210a and may be spaced apart from the first surface 210a. For example, at least a part of the insulation structure 310 may be disposed below the electronic component 301. For example, when the electronic component 301 is viewed from above (e.g., when viewed in a -z direction), the heat insulation structure 310 may at least partially overlap the electronic component 301.

For example, the heat insulation structure 310 may be formed within the third frame 230 on which the electronic component 301 is disposed. For example, the heat insulation structure 310 may be a part of the third frame 230 including air, disposed between the electronic component 301 and the first frame 210. For example, the heat insulation structure 310 may be formed on the third surface 230a facing toward the first frame 210 of the third frame 230.

For example, the at least one air gap 320 may be at least one space among the housing 200 which includes air. For example, the at least one air gap 320 may be at least one space which is sealed in the housing 200. For example, the at least one air gap 320 may separate at least a part of the electronic component 301 from the first surface 210a. For example, since the at least one air gap 320 includes air, it may have a lower thermal conductivity than a thermal conductivity of other parts distinguished from the heat insulation structure 310 in the housing 200. The at least one air gap 320 may reduce heat transfer from the electronic component 301 to the first surface 210a by being disposed between the electronic component 301 and the first surface 210a.

For example, the at least one air gap 320 may be formed on the third frame 230 on which the electronic component 301 is disposed. For example, at least one air gap 320 may be formed in a part of the third surface 230a of the third frame 230 that is disposed between the electronic component 301 and the first surface 210a. For example, the at least one air gap 320 may be sealed by the third frame 230 and the first frame 210. The insulation structure 310 may insulate heat from the electronic component 301 to the part 20 of the body of the user, which is worn on the first surface 210a, by including the at least one sealed air gap 320 in the housing 200 for reducing the heat from the electronic component 301 to the first surface 210a.

According to an embodiment, the heat insulation structure 310 may have a curvature by extending along the first surface 210a of the housing 200. For example, the first surface 210a may be bent by including a curvature in order to be worn on the part 20 (e.g., a finger) of the body of the user. The heat insulation structure 310 may be bent by extending along the bent first surface 210a. For example, since the first surface 210a forms a hole (e.g., the hole 250 in FIG. 2) for passing the part 20 of the body of the user, the heat insulation structure 310 may have a curvature by extending along the first surface 210a forming the hole 250. The heat insulation structure 310 may provide an additional space for air in the at least one air gap 320 by having a curvature.

According to an embodiment, the at least one air gap 320 may overlap the electronic component 301 when the second surface 220a is viewed from above (e.g., when viewed in the -z direction). For example, the at least one air gap 320 may face the electronic component 301. For example, the at least one air gap 320 may be disposed below the electronic component 301. For example, the electronic component 301 may be disposed above the at least one air gap 320. For example, when the electronic component 301 is viewed from above (e.g., viewed in the -z direction), the at least one air gap 320 may be covered by the electronic component 301.

According to an embodiment, the at least one air gap 320 may include a plurality of air gaps arranged along the first surface 210a of the housing 200 and spaced apart by designated intervals. For example, the heat insulation structure 310 may include a pattern in which the plurality of air gaps are spaced apart from each other by the designated intervals. The plurality of air gaps may be disposed between the electronic component 301 and the first surface 210a, respectively. As the at least one air gap 320 includes the plurality of air gaps spaced apart from each other by the designated intervals, the heat insulation structure 310 may reduce damage to the plurality of air gaps due to an external impact and provide the user with various user experiences.

According to an embodiment, the electronic device 101 may include a flexible printed circuit board 330, disposed between the first surface 210a and the second surface 220a, connected to the electronic component 301, and disposed in the housing 200. For example, the flexible printed circuit board 330 may be electrically connected to the electronic component 301. For example, the flexible printed circuit board 330 may be disposed in the housing 200. The flexible printed circuit board 330 may include a bent part corresponding to a shape of the housing 200. For example, the flexible printed circuit board 330 may be connected to a plurality of electronic components. For example, a part of the flexible printed circuit board 330 to which the electronic component 301 is coupled may be positioned above the heat insulation structure 310.

For example, the flexible printed circuit board 330 may be disposed between the first frame 210 and the second frame 220. For example, the flexible printed circuit board 330 may be disposed in the third frame 230. For example, the flexible printed circuit board 330 may include a rigid flexible printed circuit board (RFPCB), but is not limited thereto.

According to an embodiment, the electronic device 101 may further include another electronic component 302 disposed on the flexible printed circuit board 330 and disposed above the heat insulation structure 310. For example, the electronic component 301 may be referred to as a processor (e.g., the processor of FIG. 1) of the electronic device 101. The other electronic component 302 distinguished from the electronic component 301 may be referred to as a battery (e.g., the battery 189 of FIG. 1) of the electronic device 101. For example, as the electronic device 101 operates, the electronic component 301 may release heat by performing a computation. For example, as the electronic device 101 operates, the other electronic component 302 may emit heat by supplying power to the electronic device 101 and/or the electronic components in the electronic device 101. The insulation structure 310 may reduce heat transfer from the electronic component 301 and the other electronic component 302, to the part 20 of the body of the user, on which the electronic device 101 is worn, by being disposed in the housing 200 to face the electronic component 301 and the other electronic component 302.

Although the electronic device 101 has been described as including the electronic component 301, the other electronic component 302, and the heat insulation structure 310, the it is not limited thereto. The electronic device 101 may include a plurality of electronic components in the housing 200 including the electronic component 301 and the other electronic component 302. The electronic device 101 may include at least a part of the plurality of electronic components configured to emit heat and a plurality of heat insulation structures disposed between the first surface 210a of the housing 200. The plurality of heat insulation structures may reduce heat transfer from the at least a part that emits heat among the plurality of electronic components to the part 20 of the body of the electronic device 101 by respectively including the at least one air gap 320.

According to an embodiment, a sensor module 350 may be disposed between the first surface 210a and the second surface 220a. The sensor module 350 may include a light-emitting part 351 and at least one light-receiving part 352 which is spaced apart from the light-emitting part 351.

For example, the sensor module 350 may be disposed in an inner space of the housing 200 between the first surface 210a and the second surface 220a. For example, the sensor module 350 may be disposed on a component (e.g., the flexible printed circuit board 330) of the electronic device 101 between the first surface 210a and the second surface 220a. The sensor module 350 may be electrically connected to the component. For example, the sensor module 350 may be configured to sense a state of the user using the part 20 of the body of the user, which is worn on the electronic device 101. The electronic device 101 may be configured to provide the user with information related to the state through the sensed state of the user. For example, the sensor module 350 may include at least one of an optical sensor or a heartrate measurement (HRM) sensor using photoplethysmography (PPG), but is not limited thereto. The light-emitting part 351 may be referred to as a light emitting diode (LED), and the at least one light-receiving part 352 may be referred to as a photo diode, but is not limited thereto.

For example, the light-emitting part 351 may be configured to emit light in a plurality of directions. A portion of the light emitted from the light-emitting part 351 in the plurality of directions may be reflected by the part 20 of the body of the user worn on the electronic device 101. For example, the light-emitting part 351 may be configured to emit light toward the part 20 of the body of the user on which the electronic device 101 is worn. The light emitted from the light-emitting part 351 toward the part 20 of the body of the user may be reflected by the part 20 of the body. According to an embodiment, the portion of light emitted from the light-emitting part 351 in the plurality of directions may be configured to be transmitted toward the at least one light-receiving part 352.

For example, the at least one light-receiving part 352 may be configured to receive the portion of light emitted from the light-emitting part 351 in the plurality of directions. For example, the light-emitting part 351 may be configured to emit the light toward the part 20 of the body of the user, on which the electronic device 101 is worn. According to an embodiment, the at least one light-receiving part 352 may be configured to receive the portion of light reflected by the part 20 of the body of the user. For example, the at least one light-receiving part 352 may be configured such that the light-emitting part 351 receives the portion of light emitted in the plurality of directions. The at least one light-receiving part 352 may be configured such that the light-emitting part 351 receives the portion of light emitted in the plurality of directions through a space and/or a medium between the first surface 210a and the second surface 220a of the housing 200.

According to an embodiment, the sensor module 350 may be configured to sense the state of the user, based on the light emitted from the light-emitting part 351 and reflected by the part 20 of the body of the user being received by the at least one light-receiving part 352. The electronic device 101 may be configured to obtain information related to the state of the user from the sensor module 350.

According to an embodiment, the electronic device 101 may further include a processor 120 operatively coupled with the sensor module 350. For example, the electronic component 301 may be referred to as the processor 120. The processor 120 may be configured to emit light toward the part 20 of the body of the user, on which the electronic device 101 is worn, by using the light-emitting part 351 of the sensor module 350. The processor 120 may be configured to obtain biometric information on the user by receiving at least a portion of the light reflected by the part 20 of the body using the at least one light-receiving part 352 of the sensor module 350.

For example, the processor 120 may be configured to emit the light from the light-emitting part 351 of the sensor module 350 based on a user input for indicating the biometric information of the electronic device 101. The light emitted from the light-emitting unit 351 may be reflected by the part 20 of the body of the user, on which the electronic device 101 is worn. The light reflected by the part 20 of the body of the user may be received by the at least one light-receiving part 352 of the sensor module 350. The at least one light-receiving part 352 may sense the biometric information of the user through the part 20 of the body by using the light reflected by the part 20 of the body of the user. The processor 120 may be configured to provide the user with the sensed biometric information through the at least one light-receiving unit 352.

According to the above-described embodiment, the electronic device 101 may reduce heat transfer from the electronic component 301 to the part 20 of the body by including the heat insulation structure 310 between the electronic component 301 configured to emit heat and the first surface 210a facing the part 20 of the body of the user while the user wears the electronic device 101. By including the at least one air gap 320, the heat insulation structure 310 may insulate the heat from the electronic component 301 to the part 20 of the body of the user.

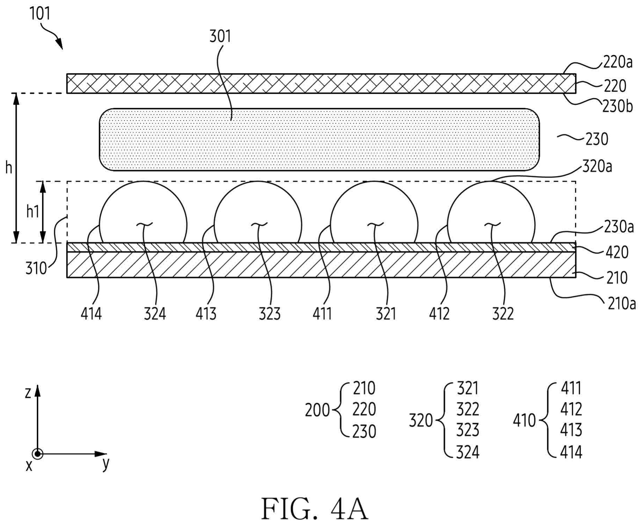

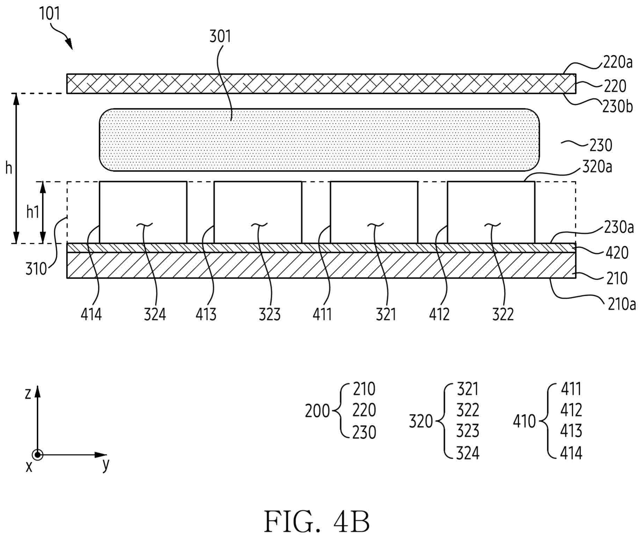

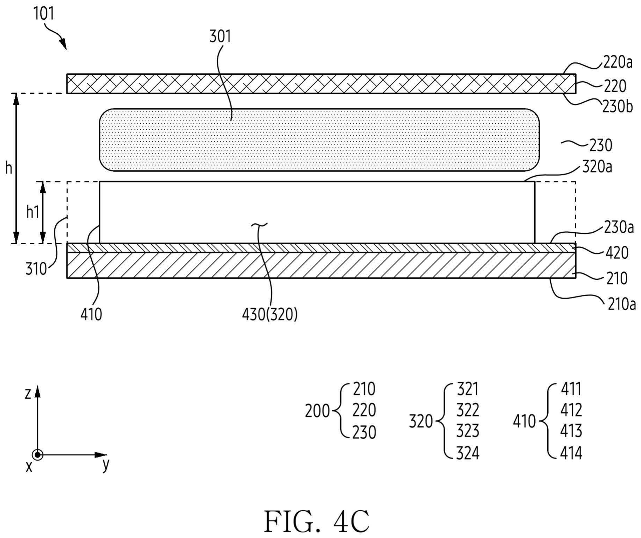

FIGS. 4A, 4B, and 4C are partial cross-sectional views of an exemplary electronic device.

Referring to FIGS. 4A, 4B, and 4C, an electronic device 101 may include a housing 200 including a first surface 210a facing a part of a body (e.g., the part 20 of the body of FIG. 2) of a user while the electronic device 101 is worn on the user, and a second surface 220a opposite the first surface 210a. The electronic device 101 may include an electronic component 301 disposed between the first surface 210a and the second surface 220a. The electronic device 101 may include a heat insulation structure 310 in the housing 200, disposed at least partially between the electronic component 301 and the first surface 210a, and including at least one air gap 320 for reducing heat transfer from the electronic component 301 to the first surface 210a. According to an embodiment, the housing 200 may further include a first frame 210 defining the first surface 210a, a second frame 220 defining the second surface 220a, and a third frame 230 interposed between the first frame 210 and the second frame 220.

According to an embodiment, the third frame 230 may include a third surface 230a facing toward the first frame 210. The heat insulation structure 310 may further include at least one groove 410 recessed from the third surface 230a toward the electronic component 301 and forming at least one air gap 320. For example, the at least one groove 410 may be formed on the third surface 230a of the third frame 230. For example, the at least one groove 410 may extend from the third surface 230a toward the electronic component 301. For example, an inner surface of the at least one groove 410 may be in contact with air. For example, the at least one groove 410 may be configured to include air in the recessed space by being recessed from the third surface 230a toward the electronic component 301. For example, the at least one groove 410 may face the electronic component 301 and may be spaced apart from the electronic component 301. For example, an end 320a of the at least one electronic component 301 adjacent to the electronic component 301 may be spaced apart from the electronic component 301. The heat insulation structure 310 may reduce heat transfer from the electronic component 301 to the part 20 of the body of the user of the electronic device 101 by including the at least one groove 410 forming the at least one air gap 320.

According to an embodiment, the heat insulation structure 310 may further include a film layer 420 in the housing 200 sealing the at least one air gap 320. The film layer 420 may be attached on the third surface 230a of the third frame 230 facing toward the first frame 210. For example, the film layer 420 may seal the at least one air gap 320 together with the third frame 230. For example, the film layer 420 may be interposed between the third surface 230a of the third frame 230 and the first frame 210. For example, the film layer 420 may be in contact with the at least one air gap 320. For example, the film layer 420 may form at least a part of the at least one air gap 320. By including the film layer 420, the heat insulation structure 310 may be configured to reduce damage to the at least one air gap 320 due to an external impact and maintain a shape of the at least one air gap 320 while the electronic device 101 is manufactured.

According to an embodiment, the film layer 420 may be omitted. In this case, the third surface 230a of the third frame 230 may be attached to the first frame 210. The heat insulation structure 310 may include the at least one groove 410 forming the at least one air gap 320 by being recessed from the third surface 230a attached to the first frame 210 toward the electronic component 301. The first frame 210 may seal the at least one air gap 320. For example, the third surface 230a may seal the at least one air gap 320 by contacting the first frame 210. For example, the at least one air gap 320 may be in contact with the first frame 210. For example, the first frame 210 may form at least a part of the at least one air gap 320. For example, the first frame 210 may be in contact with air included in the at least one air gap 320.

According to an embodiment, the at least one air gap 320 may include a plurality of air gaps 321, 322, 323, and 324 spaced apart from each other. The plurality of air gaps 321, 322, 323, and 324 may each have a curvature at a position adjacent to the electronic component 301.

For example, referring to FIG. 4A, the at least one air gap 320 may include the end 320a having a curvature at the position adjacent to the electronic component 301. For example, the at least one air gap 320 may include a shape having a curvature. For example, the inner surface of at least one groove 410 forming the at least one air gap 320 may include a shape having a curvature. For example, the shape of the at least one air gap 320 may include at least one of a spherical shape, a hemispherical shape, an oval shape, and a half oval shape, but is not limited thereto.

For example, the at least one air gap 320 may include the plurality of air gaps 321, 322, 323, and 324, respectively formed by a plurality of grooves 411, 412, 413, and 414 spaced apart from each other. The plurality of air gaps 321, 322, 323, and 324 may each have a shape having a curvature. The plurality of air gaps 321, 322, 323, and 324 may be spaced apart from each other by designated intervals. The plurality of air gaps 321, 322, 323, and 324 may form a pattern in the heat insulation structure 310 by having a curvature and substantially the same shape.

According to an embodiment, the at least one air gap 320 may include the end 320a having a flat shape at a position adjacent to the electronic component 301.

For example, when referring to FIG. 4B, the at least one air gap 320 may have the flat shape at the position adjacent to the electronic component 301. For example, the inner surface of the at least one groove 410 forming the at least one air gap 320 may include at least one plane. For example, the shape of at least one air gap 320 may include at least one of a polygonal shape, a rectangular shape, a square shape, and a trapezoid shape, but is not limited thereto.

For example, the at least one air gap 320 may include the plurality of air gaps 321, 322, 323, and 324, respectively formed by the plurality of grooves 411, 412, 413, and 414 spaced apart from each other. The plurality of air gaps 321, 322, 323, and 324 may each have the flat shape at the position adjacent to the electronic component 301. The plurality of air gaps 321, 322, 323, and 324 may be spaced apart from each other by designated intervals. The plurality of air gaps 321, 322, 323, and 324 may form another pattern different from the pattern illustrated in FIG. 4A in the heat insulation structure 310 by having substantially the same shape as each other.

According to an embodiment, the heat insulation structure 310 may further include an air layer 430 disposed between the first surface 210a and the electronic component 301 and formed by the at least one air gap 320 continuously extending along the first surface 210a. For example, referring to FIG. 4C, the air layer 430 may be formed by one air gap extending along the first surface 210a. For example, the air layer 430 may have a size corresponding to a size of the electronic component 301. For example, the air layer 430 may overlap the electronic component 301 when electronic component 301 is viewed from above (e.g., when viewed in a -z direction). For example, the air layer 430 may be sealed by the film layer 420 or the first frame 210. For example, the air layer 430 may be stacked on the film layer 420 or the first frame 210. The heat insulation structure 310 may increase insulation efficiency of the heat insulation structure 310 for heat insulation from the electronic component 301 to the part 20 of the body of the user by including the air layer 430.

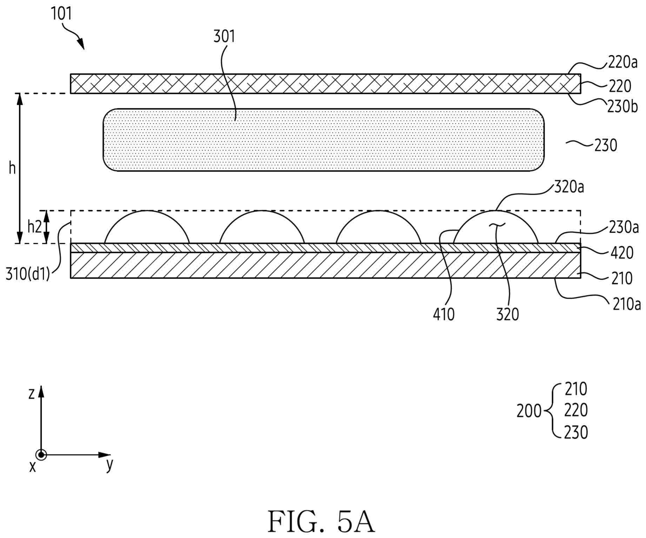

According to an embodiment, as a width h of the third frame 230 on which the electronic component 301 and the heat insulation structure 310 are disposed is changed, it is required that a length of the at least one air gap 320 in the heat insulation structure 310 from the third surface 230a toward the electronic component 301 to be changed, or the number of the at least one air gap 320 per unit volume in the heat insulation structure 310 to be changed. Regarding a change of the length of the at least one air gap 320 and a change of the number of the at least one air gap 320 per unit volume in the heat insulation structure 310, it will be described below with reference to FIGS. 5A to 5D.

According to the above-described embodiment, the heat insulation structure 310 of the electronic device 101 may increase the insulation efficiency of the heat insulation structure 310 for heat insulation from the electronic component 301 to the part 20 of the body of the user by including the plurality of air gaps 321, 322, 323, and 324 having various shapes. The at least one air gap 320 may provide various user experiences to the user by including the plurality of air gaps 321, 322, 323, and 324 forming various patterns.

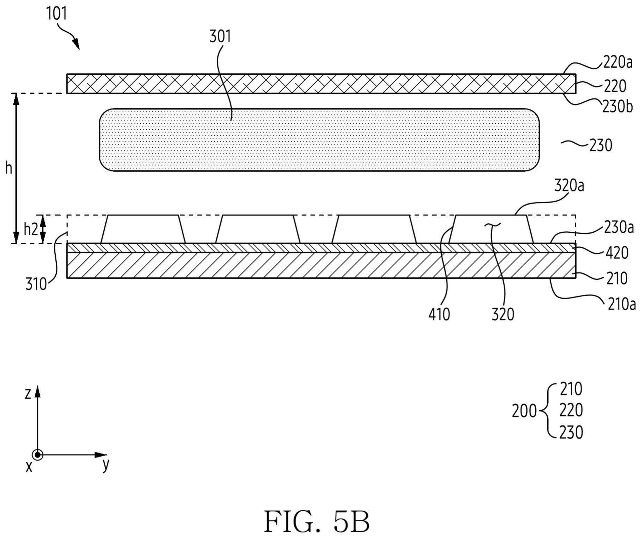

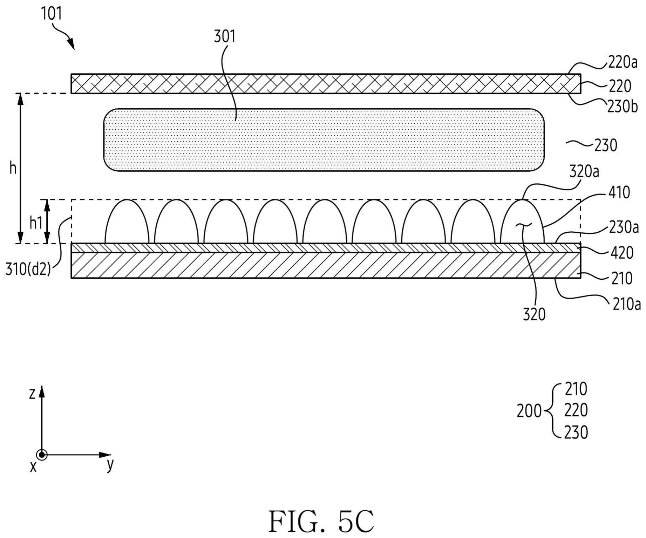

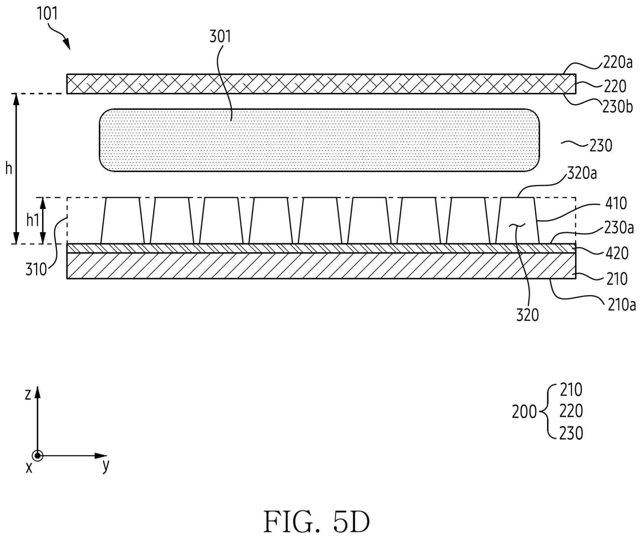

FIGS. 5A, 5B, 5C, and 5D are partial cross-sectional views of an exemplary electronic device.

Referring to FIGS. 5A, 5B, 5C, and 5D, an electronic device 101 may include a housing 200 including a first surface 210a facing a part of a body (e.g., the part 20 of the body of FIG. 2) of a user while the electronic device 101 is worn on the user, and a second surface 220a opposite the first surface 210a. The electronic device 101 may include an electronic component 301 disposed between the first surface 210a and the second surface 220a. The electronic device 101 may include a heat insulation structure 310 in the housing 200, disposed at least partially between the electronic component 301 and the first surface 210a, and including at least one air gap 320 for reducing heat transfer from the electronic component 301 to the first surface 210a. According to an embodiment, the housing 200 may further include a first frame 210 defining the first surface 210a, a second frame 220 defining the second surface 220a, and a third frame 230 interposed between the first frame 210 and the second frame 220. According to an embodiment, the heat insulation structure 310 may further include at least one groove 410 recessed from a third surface 230a toward the electronic component 301 and forming at least one air gap 320, and a film layer 420 attached to the third surface 230a and sealing the at least one groove 410.

Referring to FIGS. 5A and 5B, the at least one air gap 320 may include an end 320a adjacent to the electronic component 301. For example, when referring to FIGS. 4A and 4B together, a width h of the third frame 230 in which the electronic component 301 and the heat insulation structure 310 are disposed may be relatively small in FIGS. 5A and 5B. As the width h of the third frame 230 decreases, a length of the at least one air gap 320 in the heat insulation structure 310 from the third surface 230a toward the electronic component 301 may be adjusted from a first length h1 to a second length h2 smaller than the first length h1. The end 320a of the at least one air gap 320 may be spaced apart from the electronic component 301. As the length of the at least one air gap 320 facing the electronic component 301 is formed relatively small in the third frame 230 having the relatively small width h, the heat insulation structure 310 may reduce damage to the at least one air gap 320 and/or the third frame 230.

Referring to FIGS. 5C and 5D, the at least one air gap 320 may include a plurality of air gaps (e.g., the plurality of air gaps 321, 322, 323, and 324 of FIG. 4B). For example, when referring to FIGS. 4A and 4B together, the number of the at least one air gap 320 per unit volume in the heat insulation structure 310 may relatively increase in FIGS. 5C and 5D. As the number of the at least one air gap 320 per unit volume increases, a volume ratio of the at least one air gap 320 in the heat insulation structure 310 may increase. As the volume ratio of the at least one air gap 320 increases, air in the heat insulation structure 310 may increase. For example, as the number per unit volume of the at least one air gap 320 in the heat insulation structure 310 increases, porosity of the heat insulation structure 310 may increase. As the porosity of the heat insulation structure 310 increases, the heat insulation structure 310 may increase insulation efficiency of the heat insulation structure 310 for heat insulation from the electronic component 301 to the part 20 of the body of the user.

According to the above-described embodiment, the heat insulation structure 310 of the electronic device 101 may increase the insulation efficiency of the heat insulation structure 310 for heat insulation from the electronic component 301 to the part 20 of the body of the user and reduce damage to the housing 200 due to an external impact by including the at least one air gap 320 having various shapes.

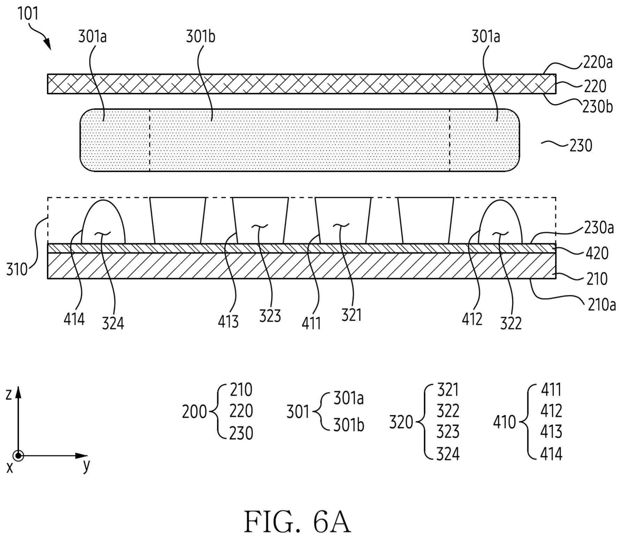

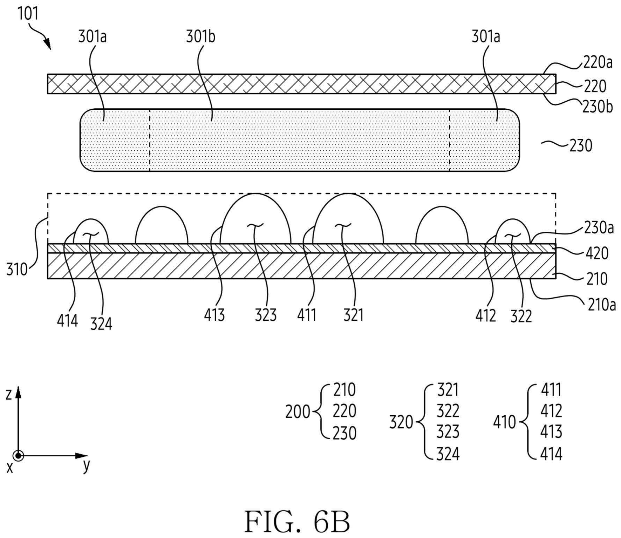

FIGS. 6A and 6B are partial cross-sectional views of an exemplary electronic device.

Referring to FIGS. 6A and 6B, an electronic device 101 may include a housing 200 including a first surface 210a facing a part of a body (e.g., the part 20 of the body of FIG. 2) of a user while the electronic device 101 is worn on the user, and a second surface 220a opposite the first surface 210a. The electronic device 101 may include an electronic component 301 disposed between the first surface 210a and the second surface 220a. The electronic device 101 may include a heat insulation structure 310 in the housing 200, disposed at least partially between the electronic component 301 and the first surface 210a, and including at least one air gap 320 for reducing heat transfer from the electronic component 301 to the first surface 210a. According to an embodiment, the housing 200 may further include a first frame 210 defining the first surface 210a, a second frame 220 defining the second surface 220a, and a third frame 230 interposed between the first frame 210 and the second frame 220.

According to an embodiment, the electronic component 301 may include a first region 301a including a periphery of the electronic component 301, and a second region 301b surrounded by the first region 301a. The at least one air gap 320 may include a first air gap 321 disposed between the second region 301b and the first surface 210a, and a second air gap 322 disposed between the first region 301a and the first surface 210a and having a volume larger than a volume of the first air gap 321.

For example, the first region 301a may be a region including both ends of the electronic component 301. For example, the first region 301a may be a portion that emits relatively little heat with respect to the second region 301b when the electronic device 101 operates. For example, the second region 301b may be a center portion of the electronic component 301. For example, the second region 301b may be a portion that emits relatively a lot of heat with respect to the first region 301a when the electronic device 101 operates.

For example, the at least one air gap 320 may include a plurality of air gaps 321, 322, 323, and 324. The heat insulation structure 310 may include at least one groove 410 forming the at least one air gap 320. For example, the plurality of air gaps 321, 322, 323, and 324 may include the first air gap 321 facing the second region 301b, and the second air gap 322 facing the first region 301a. The volume of the first air gap 321 may be larger than the volume of the second air gap 322. For example, referring to FIG. 6A, the heat insulation structure 310 may be configured such that the volume of the first air gap 321 is larger than the volume of the second air gap 322, as a shape of the first air gap 321is different from a shape of the second air gap 322. For example, referring to FIG. 6B, the heat insulation structure 310 may be configured such that the volume of the first air gap 321 is larger than the volume of the second air gap 322, as a size of the first air gap 3210 is larger than a size of the second air gap 322. The heat insulation structure 310 is configured such that an air gap facing the second part 301b of the electronic component 301 is larger than a volume of an air gap facing the first part 301a configured to emit relatively little heat with respect to the second part 301b, thereby increasing the insulation efficiency of the heat insulation structure 310 for heat insulation from the electronic component 301 to the part 20 of the body of the user.

According to the above-described embodiment, the heat insulation structure 310 of the electronic device 101 may increase the insulation efficiency of the heat insulation structure 310 for heat insulation from the electronic component 301 to the part 20 of the body of the user by including the at least one air gap 320 having various shapes.

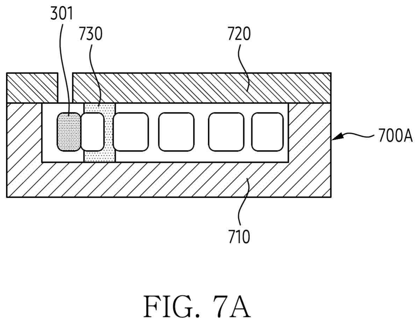

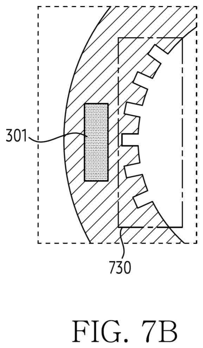

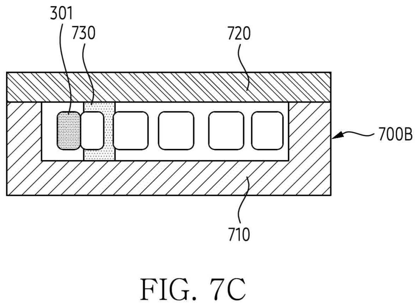

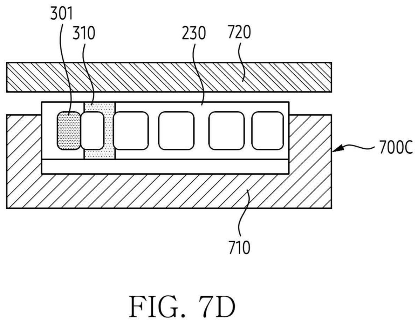

FIG. 7 illustrates a portion of a manufacturing process of an exemplary electronic device.

In step 700A, a first mold 710 and a second mold 720 for forming a third frame 230 may be prepared. The first mold 710 may include protrusions 711 protruding from the first mold 710 toward the second mold 720 for at least one air gap (e.g., the at least one air gap 320 of FIG. 3B). A plurality of electronic components including an electronic component 301 of an electronic device (e.g., the electronic device 101 of FIG. 1) may be disposed between the first mold 710 and the second mold 720. The electronic component 301 may be disposed to be positioned on the protrusions 711 of the first mold 710. A material for forming the third frame 230 may be injected between the first mold 710 and the second mold 720. The material may include at least one of epoxy, silicon, and acrylic, but is not limited thereto.

In step 700B, the material injected between the first mold 710 and the second mold 720 may be cured. For example, the material may be cured through thermal curing or ultraviolet (UV) curing. The third frame 230 may be formed through the curing of the material injected between the first mold 710 and the second mold 720. A heat insulation structure 310 in the third frame 230 may be formed through the protrusions 711 of the first mold 710.

In step 700C, the cured third frame 230 may be separated from the first mold 710 and the second mold 720. At least one groove (e.g., the at least one groove 410 of FIG. 4A) in the heat insulation structure 310 may be formed through the protrusions 711 of the first mold 710.

The third frame 230 of the electronic device 101 according to the above-described embodiment may be configured such that the heat insulation structure 310 is formed by being formed by the first mold 710 including the protrusions 711. By including the heat insulation structure 310, the third frame 230 may reduce heat transfer from the electronic component 301 to a part of a body (e.g., the part 20 of the body of FIG. 2) of a user.

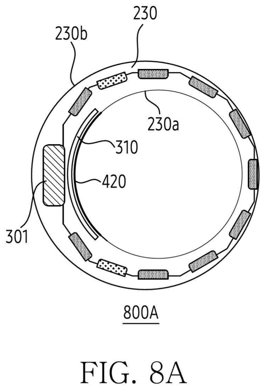

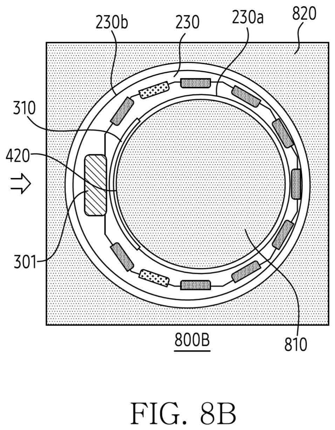

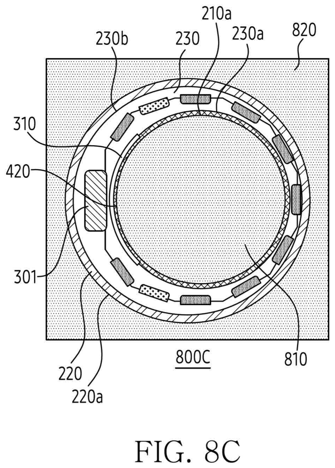

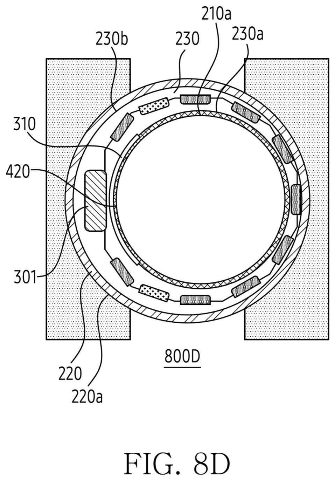

FIG. 8 illustrates a portion of a manufacturing process of an exemplary electronic device.

In step 800A, a film layer 420 may be attached on a third surface 230a of a third frame 230. The film layer 420 may be attached on at least one groove (e.g., the at least one groove 410 of FIG. 4A) in a heat insulation structure 310. As the film layer 420 is attached on the third surface 230a, at least one air gap (e.g., the at least one air gap 320 of FIG. 3B) may be formed.

In step 800B, a third mold 810 for forming a first frame 210 and a fourth mold 820 for forming a second frame 220 may be disposed. The third mold 810 may be surrounded by the third surface 230a of the third frame 230. The fourth mold 820 may surround a fourth surface 230b opposite to the third surface 230a. A material for forming the first frame 210 may be injected between the third mold 810 and the third surface 230a. A material for forming the second frame 220 may be injected between the fourth mold 820 and the fourth surface 230b. The film layer 420 may reduce damage to the at least one air gap 320 by the material for forming the first frame 210 when the material for forming the first frame 210 is injected.

In step 800C, the first frame 210 may be formed as the material for forming the first frame 210 is cured. The second frame 220 may be formed as the material for forming the second frame 220 is cured. For example, the material may be cured through thermal curing or UV curing. For example, the first frame 210 may be attached to the third surface 230a of the third frame 230. For example, the second frame 220 may be attached to the fourth surface 230b opposite to the third surface 230a of the third frame 230.

In step 800D, the third mold 810 and the fourth mold 820 may be removed. As the third mold 810 and the fourth mold 820 are removed, an electronic device (e.g., the electronic device 101 of FIG. 1) may be provided.

According to the above-described embodiment, the heat insulation structure 310 of the electronic device 101 may be formed such that the first frame 210 and the second frame 220 are attached to the third frame 230 after the film layer 420 is attached to the at least one groove 410, thereby reducing damage to the at least one air gap 320 by the first frame 210.

As described above, a wearable device (e.g., the electronic device 101 of FIG. 1) may comprise a housing (e.g., the housing 200 of FIG. 2) comprising a first surface (e.g., the first surface 210a of FIG. 2) facing a part of a body of a user while the wearable device is worn by the user, and a second surface (e.g., the second surface 220a of FIG. 2) opposite to the first surface. The wearable device may comprise an electronic component (e.g., the electronic component 301 of FIG. 3A) disposed between the first surface and the second surface. The wearable device may comprise a heat insulation structure (e.g., the heat insulation structure 310 of FIG. 3B) in the housing, disposed at least partially between the electronic component and the first surface, and including at least one air gap (e.g., at least one air gap 320 of FIG. 3B) for reducing heat transfer from the electronic component to the first surface. According to the above-mentioned embodiment, the electronic device may provide various user experiences to the user by including the first surface and the second surface opposite to the first surface. The electronic device may reduce heat transfer from the electronic component to the part of the body of the user by including the heat insulation structure. The heat insulation structure may increase insulation efficiency of the heat insulation structure by including the at least one air gap. The above-mentioned embodiment may have various effects including the above-mentioned effect.

According to an embodiment, the housing may comprise a first frame (e.g., the first frame 210 of FIG. 2) defining the first surface, a second frame (e.g., the second frame 220 of FIG. 2) defining the second surface, and a third frame (e.g., the third frame 230 of FIG. 2) interposed between the first frame and the second frame, on which the heat insulation structure is formed. According to the above-mentioned embodiment, the housing may increase wearability of the user while the user wears the electronic device by including the first frame, the second frame, and the third frame. The above-mentioned embodiment may have various effects including the above-mentioned effect.

According to an embodiment, the first frame may include at least one of metal and titanium. The third frame may include at least one of silicon, epoxy, and acryl. According to the above-mentioned embodiment, the first frame and the third frame may increase the wearability of the user while the user wears the electronic device and provide various user experiences to the user. The above-mentioned embodiment may have various effects including the above-mentioned effect.

According to an embodiment, the third frame may comprise a third surface (e.g., the third surface 230a of FIG. 3A) facing toward the first frame. The heat insulation structure may further comprise at least one groove (e.g., the at least one groove 410 of FIG. 4A) recessed from the third surface toward the electronic component and forming the at least one air gap. According to the above-mentioned embodiment, the third frame may increase insulation efficiency of the heat insulation structure by including the at least one groove forming the at least one air gap. The above-mentioned embodiment may have various effects including the above-mentioned effect.

According to an embodiment, the at least one air gap may comprise a plurality of air gaps (e.g., the plurality of air gaps 321, 322, 323, and 324 of FIG. 4) disposed along the first surface and spaced apart from each other. The plurality of air gaps may each have a curvature at a position adjacent to the electronic component. According to the above-mentioned embodiment, the at least one air gap may increase insulation efficiency of the heat insulation structure and provide various user experiences to the user by including the plurality of air gaps. The above-mentioned embodiment may have various effects including the above-mentioned effect.

According to an embodiment, the heat insulation structure may further comprise a film layer (e.g., the film layer 420 of FIG. 4A) in the housing sealing the at least one air gap. According to the above-mentioned embodiment, the heat insulation structure may reduce damage to the at least one air gap due to an external impact by further including the film layer. The above-mentioned embodiment may have various effects including the above-mentioned effect.

According to an embodiment, the at least one air gap may comprise an end (e.g., the end 320a of FIG. 4B) having a flat shape at a position adjacent to the electronic component. According to the above-mentioned embodiment, the at least one air gap may reduce damage to the at least one air gap due to an external impact by including the end having the flat shape at the position adjacent to the electronic component. The above-mentioned embodiment may have various effects including the above-mentioned effect.

According to an embodiment, the electronic component may comprise a first region (e.g., the first region 301a of FIG. 6A) including a periphery of the electronic component, and a second region (e.g., the second region 301b of FIG. 6A) surrounded by the first region. The at least one air gap may comprise a first air gap (e.g., the first air gap 321 of FIG. 6A) disposed between the second region and the first surface, and a second air gap (e.g., the second air gap 322 of FIG. 6A), disposed between the first region and the first surface, having a volume larger than a volume of the first air gap. According to the above-mentioned embodiment, the at least one air gap may increase insulation efficiency of the heat insulation structure and provide various user experiences to the user by including the first air gap and the second air gap. The above-mentioned embodiment may have various effects including the above-mentioned effect.

According to an embodiment, the at least one air gap may comprise a plurality of air gaps arranged along the first surface and spaced apart by designated intervals. According to the above-mentioned embodiment, the at least one air gap may increase insulation efficiency of the heat insulation structure and provide various user experiences to the user by including the plurality of air gaps. The above-mentioned embodiment may have various effects including the above-mentioned effect.

According to an embodiment, the heat insulation structure may have a curvature by extending along the first surface. According to the above-mentioned embodiment, the thermal insulation structure may increase insulation efficiency of the heat insulation structure heat insulation efficiency by having the curvature. The above-mentioned embodiment may have various effects including the above-mentioned effect.

According to an embodiment, the heat insulation structure may further comprise an air layer (e.g., the air layer 430 of FIG. 4C) disposed between the first surface and the electronic component and formed by the at least one air gap continuously extending along the first surface. According to the above-mentioned embodiment, the heat insulation structure may increase insulation efficiency of the heat insulation structure by including the air layer. The above-mentioned embodiment may have various effects including the above-mentioned effect.

According to an embodiment, the wearable device may further comprise a flexible printed circuit board (FPCB) (e.g., the flexible printed circuit board 330 of FIG. 3A), disposed between the first surface and the second surface, connected to the electronic component, and disposed in the housing, and another electronic component (e.g., the other electronic component 302 of FIG. 3A) disposed on the FPCB and disposed above the heat insulation structure. According to the above-mentioned embodiment, the electronic device may be configured such that the other electronic component is disposed above the heat insulation structure, thereby improving insulation efficiency of the heat insulation structure. The above-mentioned embodiment may have various effects including the above-mentioned effect.

According to an embodiment, the at least one air gap may overlap the electronic component when the second surface is viewed from above. According to the above-mentioned embodiment, the at least one air gap may increase insulation efficiency of the heat insulation structure by overlapping the electronic component. The above-mentioned embodiment may have various effects including the above-mentioned effect.

According to an embodiment, the housing may further comprise a first frame forming the first surface, a second frame forming the second surface, and a third frame disposed between the first frame and the second frame, in which the electronic component is disposed, the third frame including a third surface attached to the first frame and a fourth surface attached to the second frame. The heat insulation structure may further comprise at least one groove recessed from the third surface attached to the first frame toward the electronic component and forming the at least one air gap. The first frame may seal the at least one air gap. According to the above-mentioned embodiment, the third frame may be configured such that the third surface is attached to the first frame, thereby sealing the at least one air gap. The above-mentioned embodiment may have various effects including the above-mentioned effect.

According to an embodiment, the wearable device may further comprise a sensor module (e.g., the sensor module 350 of FIG. 3A), disposed between the first surface and the second surface, including a light-emitting part (e.g., the light-emitting part 351 of FIG. 3A) and a light-receiving part (e.g., the at least one light-receiving part 352 of FIG. 3A) spaced apart from the light-emitting part. The sensor module may be configured to emit light toward the part of the body of the user. The sensor module may be configured to obtain biometric information about the user based on receiving at least a portion of the light reflected by the part of the body of the user through the light-receiving part. According to the above-mentioned embodiment, the wearable device may provide various user experiences to the user by including the sensor module. The above-mentioned embodiment may have various effects including the above-mentioned effect.

According to an embodiment, a wearable device may comprise a housing comprising a first frame including a first surface facing a part of a body of a user while the wearable device is worn by the user, a second frame including a second surface opposite to the first surface, and a third frame disposed between the first frame and the second frame. The wearable device may comprise an electronic component disposed in the third frame. The third frame may comprise a third surface facing toward the first frame. The third frame may comprise a heat insulation structure, disposed at least partially between the electronic component and the first frame, and including at least one groove recessed from the third surface toward the electronic component, and at least one air gap formed by the at least one groove for reducing heat transfer from the electronic component to the first surface. According to the above-mentioned embodiment, the electronic device may provide various user experiences to the user by including the first surface and the second surface opposite to the first surface. The electronic device may reduce heat transfer from the electronic component to the part of the body of the user by including the heat insulation structure. The heat insulation structure may increase insulation efficiency of the heat insulation structure by including the at least one air gap. The above-mentioned embodiment may have various effects including the above-mentioned effect.

According to an embodiment, the heat insulation structure may further comprise a film layer attached on the third surface and sealing the at least one air gap. According to the above-mentioned embodiment, the heat insulation structure may reduce damage to the at least one air gap due to an external impact by further including the film layer. The above-mentioned embodiment may have various effects including the above-mentioned effect.

According to an embodiment, the wearable device may further comprise a FPCB, disposed between the first surface and the second surface, connected to the electronic component, and disposed in the housing, and another electronic component disposed on the FPCB and disposed above the heat insulation structure. According to the above-mentioned embodiment, the electronic device may be configured such that the other electronic component is disposed above the heat insulation structure, thereby improving insulation efficiency of the heat insulation structure. The above-mentioned embodiment may have various effects including the above-mentioned effect.

According to an embodiment, the at least one air gap may include a plurality of air gaps disposed along the first surface and spaced apart from each other. The plurality of air gaps may each have a curvature at a position adjacent to the electronic component. According to the above-mentioned embodiment, the at least one air gap may increase insulation efficiency of the heat insulation structure and provide various user experiences to the user by including the plurality of air gaps. The above-mentioned embodiment may have various effects including the above-mentioned effect.

According to an embodiment, the first frame may include at least one of metal and titanium. The third frame may include at least one of silicon, epoxy, and acryl. According to the above-mentioned embodiment, the first frame and the third frame may increase the wearability of the user while the user wears the electronic device and provide various user experiences to the user. The above-mentioned embodiment may have various effects including the above-mentioned effect.

The electronic device according to various embodiments may be one of various types of electronic devices. The electronic devices may include, for example, a portable communication device (e.g., a smartphone), a computer device, a portable multimedia device, a portable medical device, a camera, a wearable device, or a home appliance. According to an embodiment of the disclosure, the electronic devices are not limited to those described above.

It should be appreciated that various embodiments of the present disclosure and the terms used therein are not intended to limit the technological features set forth herein to particular embodiments and include various changes, equivalents, or replacements for a corresponding embodiment. With regard to the description of the drawings, similar reference numerals may be used to refer to similar or related elements. It is to be understood that a singular form of a noun corresponding to an item may include one or more of the things unless the relevant context clearly indicates otherwise. As used herein, each of such phrases as "A or B," "at least one of A and B," "at least one of A or B," "A, B, or C," "at least one of A, B, and C," and "at least one of A, B, or C," may include any one of or all possible combinations of the items enumerated together in a corresponding one of the phrases. As used herein, such terms as "1st" and "2nd," or "first" and "second" may be used to simply distinguish a corresponding component from another, and does not limit the components in other aspect (e.g., importance or order). It is to be understood that if an element (e.g., a first element) is referred to, with or without the term "operatively" or "communicatively", as "coupled with," or "connected with" another element (e.g., a second element), it means that the element may be coupled with the other element directly (e.g., wiredly), wirelessly, or via a third element.

As used in connection with various embodiments of the disclosure, the term "module" may include a unit implemented in hardware, software, or firmware, and may interchangeably be used with other terms, for example, "logic," "logic block," "part," or "circuitry". A module may be a single integral component, or a minimum unit or part thereof, adapted to perform one or more functions. For example, according to an embodiment, the module may be implemented in a form of an application-specific integrated circuit (ASIC).

Various embodiments as set forth herein may be implemented as software (e.g., the program 140) including one or more instructions that are stored in a storage medium (e.g., internal memory 136 or external memory 138) that is readable by a machine (e.g., the electronic device 101). For example, a processor (e.g., the processor 120) of the machine (e.g., the electronic device 101) may invoke at least one of the one or more instructions stored in the storage medium, and execute it, with or without using one or more other components under the control of the processor. This allows the machine to be operated to perform at least one function according to the at least one instruction invoked. The one or more instructions may include a code generated by a complier or a code executable by an interpreter. The machine-readable storage medium may be provided in the form of a non-transitory storage medium. Wherein, the term "non-transitory" simply means that the storage medium is a tangible device, and does not include a signal (e.g., an electromagnetic wave), but this term does not differentiate between a case in which data is semi-permanently stored in the storage medium and a case in which the data is temporarily stored in the storage medium.

According to an embodiment, a method according to various embodiments of the disclosure may be included and provided in a computer program product. The computer program product may be traded as a product between a seller and a buyer. The computer program product may be distributed in the form of a machine-readable storage medium (e.g., compact disc read only memory (CD-ROM)), or be distributed (e.g., downloaded or uploaded) online via an application store (e.g., PlayStore^{™}), or between two user devices (e.g., smart phones) directly. If distributed online, at least part of the computer program product may be temporarily generated or at least temporarily stored in the machine-readable storage medium, such as memory of the manufacturer's server, a server of the application store, or a relay server.

According to various embodiments, each component (e.g., a module or a program) of the above-described components may include a single entity or multiple entities, and some of the multiple entities may be separately disposed in different components. According to various embodiments, one or more of the above-described components may be omitted, or one or more other components may be added. Alternatively or additionally, a plurality of components (e.g., modules or programs) may be integrated into a single component. In such a case, according to various embodiments, the integrated component may still perform one or more functions of each of the plurality of components in the same or similar manner as they are performed by a corresponding one of the plurality of components before the integration. According to various embodiments, operations performed by the module, the program, or another component may be carried out sequentially, in parallel, repeatedly, or heuristically, or one or more of the operations may be executed in a different order or omitted, or one or more other operations may be added.

## Claims

1. A wearable device (101) comprising:
a housing (200) comprising a first surface (210a) facing a part of a body of a user while the wearable device (101) is worn by the user, and a second surface (210b) opposite to the first surface (210a);
an electronic component (301) disposed between the first surface (210a) and the second surface (210b); and
a heat insulation structure (310) in the housing (200), disposed at least partially between the electronic component (301) and the first surface (210a), and including at least one air gap (320) for reducing heat transfer from the electronic component (301) to the first surface (210a).

2. The wearable device (101) of claim 1,
wherein the housing (200) comprises:
a first frame (210) defining the first surface (210a),
a second frame (220) defining the second surface (210b), and
a third frame (230) interposed between the first frame (210) and the second frame (220), on which the heat insulation structure (310) is formed,

3. The wearable device (101) of claim 1 or 2,
wherein the second frame (220) includes at least one of metal and titanium, and
wherein the third frame (230) includes at least one of silicon, epoxy, and acryl.

4. The wearable device (101) of any one of claims 1 to 3,
wherein the third frame (230) comprises a third surface (230a) facing toward the first frame (210), and
wherein the heat insulation structure (310) further comprises at least one groove (410) recessed from the third surface (230a) toward the electronic component (301) and forming the at least one air gap (320).

5. The wearable device (101) of any one of claims 1 to 4,
wherein the at least one air gap (320) comprises a plurality of air gaps (321, 322, 323, 324) spaced apart from each other, and
wherein the plurality of air gaps (321, 322, 323, 324) each have a curvature at a position adjacent to the electronic component (301).

6. The wearable device (101) of any one of claims 1 to 5,
wherein the heat insulation structure (310) further comprises a film layer (420) in the housing (200) sealing the at least one air gap (320).

7. The wearable device (101) of any one of claims 1 to 6,
wherein the at least one air gap (320) comprises an end (320a) having a flat shape at a position adjacent to the electronic component (301).

8. The wearable device (101) of any one of claims 1 to 7
wherein the electronic component (301) comprises:
a first region (301a) including a periphery of the electronic component (301), and
a second region (301b) surrounded by the first region (301a), and
wherein the at least one air gap (320) comprises:
a first air gap (321) disposed between the second region (301b) and the first surface (210a), and
a second air gap (322), disposed between the first region (301a) and the first surface (210a), having a volume larger than a volume of the first air gap (321).

9. The wearable device (101) of any one of claims 1 to 8,
wherein the at least one air gap (320) comprises a plurality of air gaps (321, 322, 323, 324) arranged along the first surface (210a) and spaced apart by designated intervals.

10. The wearable device (101) of any one of claims 1 to 9,
wherein the heat insulation structure (310) has a curvature by extending along the first surface (210a).

11. The wearable device (101) of any one of claims 1 to 10,
wherein the heat insulation structure (310) further comprises an air layer (430) disposed between the first surface (210a) and the electronic component (301) and formed by the at least one air gap (320) continuously extending along the first surface (210a).

12. The wearable device (101) of any one of claims 1 to 11, further comprising:
a flexible printed circuit board (FPCB) (330), disposed between the first surface (210a) and the second surface (210b), connected to the electronic component (301), and disposed in the housing (200); and
another electronic component (302) disposed on the FPCB (330) and disposed above the heat insulation structure (310).

13. The wearable device (101) of any one of claims 1 to 12,
wherein the at least one air gap (320) overlaps the electronic component (301) when the second surface (210b) is viewed from above.

14. The wearable device (101) of any one of claims 1 to 13,
wherein the housing (200) further comprises:
a first frame (210) forming the first surface (210a),
a second frame (220) forming the second surface (210b), and
a third frame (230) disposed between the first frame (210) and the second frame (220), in which the electronic component (301) is disposed, the third frame (230) including a third surface (230a) attached to the first frame (210) and a fourth surface attached to the second frame (220),
wherein the heat insulation structure (310) further comprises at least one groove (410) recessed from the third surface (230a) attached to the first frame (210) toward the electronic component (301) and forming the at least one air gap (320), and
wherein the first frame (210) seals the at least one air gap (320).

15. The wearable device (101) of any one of claims 1 to 14, further comprising:
a sensor module (350), disposed between the first surface (210a) and the second surface (210b), including a light-emitting part (351) and a light-receiving part (352) spaced apart from the light-emitting part (351),
wherein the sensor module (350) is configured to:
emit light toward the part of the body of the user, and
obtain biometric information about the user based on receiving at least a portion of the light reflected by the part of the body of the user through the light-receiving part (352).
